Europäisches Patentamt

European Patent Office

Office européen des brevets

(19)

(11) EP 0 150 735 B2

(12) NEW EUROPEAN PATENT SPECIFICATION

(45) Date of publication and mention
of the opposition decision:
07.01.2004 Bulletin 2004/02

(45) Mention of the grant of the patent:
29.07.1992 Bulletin 1992/31

(21) Application number: 85100223.8

(22) Date of filing: 11.01.1985

(51) Int Cl.7: **C12P 21/00**, C12P 21/02,
C12Q 1/68, C07K 14/00

(54) **Protein composition exhibiting coagulation activity and method for the preparation thereof**

Proteinzusammensetzung mit Koagulations-wirkung und Verfahren zu ihrer Herstellung

Composition protéique à activité coagulante et méthode de préparation de ladite composition

(84) Designated Contracting States:
**AT BE CH DE FR GB IT LI LU NL SE**

(30) Priority: **12.01.1984 US 570062**
**26.10.1984 US 664919**

(43) Date of publication of application:
**07.08.1985 Bulletin 1985/32**

(60) Divisional application:
**91113267.8 / 0 466 199**

(73) Proprietors:
• **CHIRON CORPORATION**
**Emeryville, California 94608 (US)**
• **NOVO NORDISK A/S**
**2880 Bagsvaerd (DK)**

(72) Inventors:
• **Kuo, George**
**San Francisco California 94122 (US)**
• **Masiarz, Frank R.**
**San Francisco California 94131 (US)**
• **Truett, Martha**
**Oakland California 94611 (US)**
• **Valenzuela, Pablo**
**San Francisco California 94117 (US)**
• **Rasmussen, Mirella Ezban**
**DK-2100 Copenhagen (DK)**
• **Favaloro, Jennifer**
**Carlton 3035 Victoria (AU)**

(74) Representative: **Hallybone, Huw George et al**
**Carpmaels and Ransford,**
**43 Bloomsbury Square**
**London WC1A 2RA (GB)**

(56) References cited:
EP-A- 0 123 945    EP-A- 0 157 556
EP-A- 0 160 457    EP-A- 0 182 372
WO-A-84/04541    WO-A-85/01961

• **CHEMICAL ABSTRACTS, vol. 98, no. 21, 23rd
May 1983, page 450, abstract no. 176896j,
Columbus, Ohio, US; C.A. FULCHER et al.:
"Thrombin proteolysis of purified factor VIII
procoagulant protein: correlation of activation
with generation of a specific polypeptide", &
BLOOD 1983, 61(4), 807-11**
• **PROC.NATL.ACAD.SCI.USA, vol. 79, March
1982, pages 1648-1652, Scripps Clinic and
Research Foundation, La Jolla, California, US;
C.A. FULCHER et al.: "Characterization of the
human factor VIII procoagulant protein with a
heterologous precipitating antibody"**
• **PROC.NATL.ACAD.SCI.USA, vol. 79, November
1982, pages 6461-6464, University of
Washington, Seattle, US; K. KURACHI et al.:
"Isolation and characterization of a cDNA coding
for human factor IX"**
• **MOLECULAR CLONING, A LABORATORY
MANUAL, 10th November 1982, pages 224-228,
Cold Spring Harbour Laboratory, US; T.
MANIATIS et al.: "Strategies for cDNA cloning"**
• **NATURE, vol. 303, 9th June 1983, pages 474-475,
Macmillan Journals Ltd, GB; A.L. BLOOM:
"Benefits of cloning genes for clotting factors"**
• **NATURE, vol. 306, no. 5943, 8th December 1983,
page 528, Macmillan Journals Ltd, GB; J.
MADDOX: "Factor VIII cloning"**
• **BIOLOGICAL ABSTRACTS, vol. 77, no. 6, March
1984, page 4443, abstract no. 40713,
Philadelphia, PA, US; F. ROTBLAT et al.:
"Monoclonal antibodies to human procoagulant
factor VIII-VIII:C", & J LAB CLIN MED 101(5):
736-746. 1983**

EP 0 150 735 B2

- **CHEMICAL ABSTRACTS, vol. 98, no. 21, 23rd May 1983, page 450, abstract no. 176896j, Columbus, Ohio, US; C.A. FULCHER et al.: "Thrombin proteolysis of purified factor VIII procoagulant protein: correlation of activation with generation of a specific polypeptide", & BLOOD 1983, 61 (4), 807-11**
- **NATURE, vol. 312, no. 5992, 22nd November 1984, pages 330-337, Reading, Berks, GB; W.I. WOOD et al.: "Expression of active human factor VIII from recombinant DNA clones"**
- **NATURE, vol. 312, no. 5992, 22nd November 1984, pages 342-347, Reading, Berks, GB; J.J. TOOLE et al.: "Molecular cloning of a cDNA encoding human antihaemophilic factor"**

Remarks:
   Divisional application 91113267.8 filed on 11/01/85.

**Description**

**Description for the following Contracting States : BE, CH, DE, FR, GB, IT, LI, LU, NL, SE**

The invention relates to a novel protein composition comprising a calcium bridged complex of two polypeptide fragments, exhibiting a coagulation activity similar to that of human Factor VIIIC, and a method for the preparation thereof.

Factor VIIIC is a plasma protein that participates in the intrinsic pathway of blood coagulation. It is absent or defective in individuals with the hereditary X chromosome-linked recessive bleeding disorder hemophilia A. Great difficulty has been encountered in isolating Factor VIIIC due to its extremely low concentration in plasma and the fact that it appears to be an intermediate or final degradation product of a larger protein precursor. Therefore, efforts to isolate Factor VIIIC have led to complex mixtures of significant heterogeneity and varying molecular weights.

One of the approaches which has found broad application to the production of physiologically active proteins involves the isolation of the protein of interest in purified form. The protein of interest provides invaluable aid in the development of a recombinant DNA capability for the production of the protein. By having the protein of interest, one may prepare monoclonal antibodies which are specific for the protein and can be used to establish the production of the protein in lysates, by means of expression from messenger RNA in oocytes, or from a cDNA gene in unicellular microorganisms. In addition, by amino acid sequencing, one can develop probes, employing codons coding for the particular amino acid sequence, for hybridization to messenger RNA, chromosomal DNA or cDNA and, therefore, provide for the detection, isolation and expression of the relevant gene or message and the production of the desired product in high yields in one or more hosts.

US-A-4 361 509 and references cited therein describe purification of Factor VIIIC (see also Fulcher and Zimmerman, Proc. Natl. Acad. Sci. USA (1982) 79, 1648-1652). Tuddenham et al., J. of Lab. Clinical Medicine (1979) 93, 40-53 describe purification of Factor VIIIC using polyclonal antibodies. Austen, British J. Hematology (1979) 43, 669-674 describes the use of aminohexyl-Sepharose for Factor VIIIC purification. Weinstein et al., Proc. Natl. Acad. Sci. USA (1981) 78, 5137-5141 describe a study of the effect of thrombin on Factor VIIIC. See also Kuo et al., Abstracts for IX International Congress of Thrombosis and Hemostasis, (Copenhagen; July, 1983).

It has further been known from C.A. Fulcher, J.R. Roberts and T.S. Zimmerman, Blood (1983) 61, No. 4, 807-811, that two polypeptide fragments of 79/80 kd and 92 kd can be isolated from the thrombin degradation product of human Factor VIIIC by SDS gel electrophoresis.

It is the object of the present invention to provide a protein composition exhibiting a coagulation activity similar to that of human Factor VIIIC and a method for the preparation thereof.

The above object is achieved according to the claim.

The protein composition of the present invention is an isolated protein composition comprising a complex of a 77/80 kd doublet polypeptide fragment calcium bridged with a 92.5 kd polypeptide fragment; and precursor species said complex exhibiting a coagulation activity similar to that of human Factor VIIIC, wherein said complex and precursor species have a purity of at least 90% based on total protein, and wherein the complex has a purity of at least 30% based on total protein.

**Description for the following Contracting State : AT**

The invention relates to a method for the preparation of a novel protein composition comprising a calcium bridged complex of two polypeptide fragments, exhibiting a coagulation activity similar to that of human Factor VIIIC.

Factor VIIIC is a plasma protein that participates in the intrinsic pathway of blood coagulation. It is absent or defective in individuals with the hereditary X chromosome-linked recessive bleeding disorder hemophilia A. Great difficulty has been encountered in isolating Factor VIIIC due to its extremely low concentration in plasma and the fact that it appears to be an intermediate or final degradation product of a larger protein precursor. Therefore, efforts to isolate Factor VIIIC have led to complex mixtures of significant heterogeneity and varying molecular weights.

One of the approaches which has found broad application to the production of physiologically active proteins involves the isolation of the protein of interest in purified form. The protein of interest provides invaluable aid in the development of a recombinant DNA capability for the production of the protein. By having the protein of interest, one may prepare monoclonal antibodies which are specific for the protein and can be used to establish the production of the protein in lysates, by means of expression from messenger RNA in oocytes, or from a cDNA gene in unicellular microorganisms. In addition, by amino acid sequencing, one can develop probes, employing codons coding for the particular amino acid sequence, for hybridization to messenger RNA, chromosomal DNA or cDNA and, therefore, provide for the detection, isolation and expression of the relevant gene or message and the production of the desired product in high yields in one or more hosts.

US-A-4 361 509 and references cited therein describe purification of Factor VIIIC (see also Fulcher and Zimmerman, Proc. Natl. Acad. Sci. USA. (1982) 79, 1648-1652). Tuddenham et al., J. of Lab. Clinical Medicine (1979) 93, 40-53 describe purification of Factor VIIIC using polyclonal antibodies. Austen, British J. Hematology (1979) 43, 669-674 describes the use of aminohexyl-Sepharose for Factor VIIIC purification. Weinstein et al., Proc. Natl. Acad. Sci. USA (1981) 78, 5137-5141 describe a study of the effect of thrombin on Factor VIIIC. See also Kuo et al., Abstracts for IX International Congress of Thrombosis and Hemostasis, (Copenhagen; July, 1983).

It has further been known from C.A. Fulcher, J.R. Roberts and T.S. Zimmerman, Blood (1983) 61, No. 4, 807-811, that two polypeptide fragments of 79/80 kd and 92 kd can be isolated from the thrombin degradation product of human Factor VIIIC by SDS gel electrophoresis.

It is the object of the present invention to provide a method for the preparation of a protein composition exhibiting a coagulation activity similar to that of human Factor VIIIC.

The above object is achieved according to the claim.

The protein composition concerned is an isolated protein composition comprising a complex of a 77/80 kd doublet polypeptide fragment calcium bridged with a 92.5 kd polypeptide fragment, and precursor species said complex exhibiting a coagulation activity similar to that of human Factor VIIIC, wherein said complex and precursor species have a purity of at least 90% based on total protein, and wherein the complex has a purity of at least 30% based on total protein.


Continuation of the Descriptions valid for all cited Contracting States


The method for preparing that protein composition of the present invention comprises the steps of

(A) subjecting human Factor VIIIC to immunosorbent chromatography using a polyclonal anti-VIIIR-Sepharose column,
(B) separating the resulting material on an aminohexyl-substituted agarose column, and
(C) further chromatographic purification.


The human Factor VIIIC fragments and subunits are provided in substantially pure form. The polypeptide fragments of the above-defined protein composition may be used for numerous purposes, e.g. for production of human Factor VIIIC, precursors and subunits thereof, by means of expression in a microorganism or mammalian tissue culture cells. Within the scope of the invention, purified Factor VIIIC, subunits and fragments thereof have been isolated, and their physiological relationship has been determined, particularly employing thrombin digestion. At least a portion of each of the related series of polypeptides may be sequenced, and the sequences may be employed for developing complex probes. Further, genomic DNA fragments may be probed for homologous sequences, and hybridizing fragments may be isolated and further manipulated to provide a DNA fragment encoding a complete subunit or fragment, and/or used for isolating mature mRNA, from which ds cDNA may be obtained. The DNA sequence may then be further manipulated for insertion into an expression vector and the expression vector employed for introduction into a compatible host for expression of the polypeptide.

In addition, the compositions maybe used for the expression of Factor VIIIC subunits and fragments for producing Factor VIIIC as a precursor or in its active form or providing individual subunits for use in combination with naturally available subunits. The subunits and fragments have one or more biological properties associated with Factor VIIIC, such as epitopic sites, coagulation activity, and immunogenicity, so as to be used for producing antibodies which find use as reagents, particularly labeled reagents in immunoassays.

Human Factor VIIIC is a complex protein which can be isolated in substantially pure form exhibiting an apparent molecular weight of about 460 kd. Upon electrophoresis under denaturing conditions, a large number of fragments result of varying molecular weights: 240, 160, 140, 115, 92.5, 80 and 77 kd, the latter two being found to migrate as a doublet. Analysis of the fragments by chemical and protease cleavage including thrombin, employing antibodies to follow immunogenic relationships and cleavage patterns to follow structural relationships, demonstrates that the 92.5 kd polypeptide is related to the 240, 160, 140 and 115 polypeptides while the 77/80 kd doublet may not have common identifiable characteristics with the other peptides, except for the 240 kd polypeptide. It is further found that the 77/80 kd doublet is converted by thrombin to a 67/70 kd doublet, while the 92.5 kd polypeptide present in purified Factor VIIIC material treated with thrombin is cleaved by thrombin, directly or indirectly, into two polypeptides of about 40 and 52.5 kd. It is found that the electrophoretically isolated 77/80 kd doublet polypeptides have their N-termini blocked, while the 67/70 kd doublet polypeptides do not have their N-termini blocked.

It is further found that the locus for Factor VIIIC involves exons with large introns, where exons involve various domains associated with Factor VIIIC. Thus, individual exons can be isolated which involve specific polypeptides or fragments thereof involved with the Factor VIIIC complex. By identifying specific amino acid sequences involved with Factor VIIIC subunits and fragments thereof, one can selectively isolate the exons from genomic DNA and use the exons by themselves, in combination, or joined by synthetic DNA to provide for sequences encoding for and production of polypeptide subunits of Factor VIIIC or fragments thereof.

Conveniently, the Factor VIIIC genomic DNA sequences containing both exons and introns may be inserted into an expression vector appropriate for transcription and translation in mammalian cells to provide for both substantial quantities of properly spliced messenger RNA suitable for cDNA cloning and production of Factor VIIIC, subunits or fragments. In addition, the DNA sequences isolated from the genome can be used for hybridizing to natural messenger RNA encoding for Factor VIIIC. The messenger RNA may then be used to prepare ds cDNA for encoding the subunits of Factor VIIIC. The DNA sequences may be employed for expression by insertion into an appropriate expression vector having the necessary regulatory signals for transcription and translation. The Factor VIIIC gene expression vector (an expression vector carrying one or more genes encoding for all or a portion of Factor VIIIC, precursor, subunits or fragments thereof) may be introduced into a compatible host and the host grown for expression of Factor VIIIC. By appropriate choice of hosts, the Factor VIIIC DNA may be inserted downstream from a secretory leader and processing signals, so that the product will be secreted from the host and processed to provide for the complete polypeptide. As appropriate, the polypeptide may be further processed to introduce functionalities or substituents present on the naturally occurring polypeptide.

The divisional EP application 91113267.8 relates to a process for the synthesis of polypeptide fragments of human Factor VIIIC, particularly a fragment having a molecular weight of 77/80 kd, DNA sequences encoding portions of this polypeptide fragment, extrachromosomal elements comprising these DNA fragments, a complex probe for screening DNA sequences, corresponding DNA constructs, and monoclonal antibodies prepared with an immunogen comprising the above polypeptide fragments.

In the first stage of the method used in the subject invention, highly purified Factor VIIIC is obtained and characterized. Purified Factor VIIIC can be obtained from commercially available human anti-hemophilic factor (AHF), which is prepared from fresh, normal human plasma as a cryoprecipitate and represents about a 40-fold enrichment. The Factor VIIIC is further concentrated and purified by dissolving the anti-hemophilic factor in an appropriate buffer, e.g., saline imidazole-lysine-HCl, pH 7.4, followed by chromatography on an affinity column having either polyclonal or monoclonal antibodies to Factor VIIIC or Factor VIIIR linked thereto. Conveniently, the antibodies are covalently bonded to a Sepharose support. Factor VIIIC may be eluted from the column employing a combination of a relatively high concentration of calcium ion with glycerol. The fractions obtained from the column may then be dialysed with an appropriate buffer, as described above, containing a low concentration of calcium ion, and are then further purified employing an aminohexyl-Sepharose column eluted with a high calcium or sodium chloride concentration buffer. Additional chromatographic steps, using; e.g., gelation Sepharose, HPLC, ion exchange on dextran sulfate or Mono Q, affinity columns using lectins or antibodies to Factor VIIIC, provide additional purification. Particularly, the use of dextran sulfate removes trace contamination, e.g., fibrinogen, fibronectin, IgG, from the preparation, so as to leave a product substantially free of foreign proteins. The activity of the fractions from the columns may be monitored for either or both biological and antigenic activity using coagulation assay (commercially available kits) and antibodies specific for Factor VIIIC. Based on the concentration of Factor VIII in plasma, purifications of about 200,000-fold may be achieved by the above-described method.

Characterization of Factor VIIIC

Gel filtration indicates that Factor VIIIC behaves as a complex with an apparent molecular weight of about 460 kd. Using SDS-gel electrophoresis (denaturing conditions), seven individual polypeptides can be isolated of differing molecular weight. The fragments as defined by their molecular weight are 240, 160, 140, 115, 92.5, 80 and 77 kd. These fragments were characterized in the following ways.

The first study involved employing inhibitor antibodies isolated from hemophilic patients, the antibodies being designated as Z and E. Both antibodies reacted with the 77/80 kd doublet. The E antibody reacted strongly with the 240 kd polypeptide and weakly with several bands between the doublet and the 240 kd polypeptide. The Z antibody also reacted weakly with the 240 kd polypeptide.

In immunoprecipitation experiments, the E antibody precipitates the 77/80 kd doublet as well as the high molecular weight species of 160, 140, 115 and 92.5 kd, with the doublet among the stronger bands. Inclusion of EGTA results in the loss of the bands other than the doublet indicating that the 92.5 kd species is associated with the 77 kd and/or 80 kd species in a complex mediated by a $Ca^{++}$ bridge.

In the next study, monoclonal antibodies were prepared, which both inhibit Factor VIIIC-mediated coagulation activity and react with components of the complex: Class I reacting with the 77/80 kd doublet and 240 kd polypeptides; Class II reacting with the 160, 140, 115 and 92.5 kd polypeptides. Immunoprecipitation of thrombin-digested Factor VIIIC with Class I antibodies indicates that the 70/67 kd doublet which results is derived from the 77/80 kd doublet present in Factor VIIIC. The Class II monoclonals indicated that the 160, 140 and 115 kd peptides are precursors of the 92.5 kd peptide. A 40 kd peptide cleavage product of 92.5 kd peptide was also bound by the Class II antibodies. An ELISA assay using monoclonal antibodies in the presence and absence of EGTA confirms the $Ca^{++}$ bridge association between the 92.5 kd and 77 kd and/or 80 kd components of the Factor VIIIC complex.

Both the human inhibitor and monoclonal antibodies may be used in immunosorbent column chromatography procedures to obtain Factor VIIIC or using EGTA, to resolve its constituent components, the 92.5 kd and 77/80 kd species.

The next study involved thrombin degradation of purified Factor VIIIC material at pH 6.8 or 7.4. Aliquots were assayed for coagulation activity and TCA-precipitated for gel analysis. Coagulation activity was shown to increase with time and then decrease coincidently with an increase and decrease in the amount of the 92.5 kd species. Thrombin treatment of the purified Factor VIIIC material for short periods of time (5-15 min) enhances the amount of 92.5 kd species, while the 77/80 kd doublet is partially converted into a 67/70 kd doublet. When long thrombin digestion times are employed, e.g., one hour, the 92.5 kd protein is degraded, and two new peptides of 40 and 52.5 kd appear, with the 40 kd peptide retaining immunogenic characteristics of the 92.5 kd species. The 52.5 kd peptide is shown to be a cleavage product by chemical and enzymatic degradation patterns and products analogous to the 92.5 kd species.

In the next study individual Factor VIIIC subunits and precursors (e.g., 240, 77/80, 92.5 kd species) were isolated by preparative SDS gel electrophoresis, and a time course thrombin digestion of the isolated polypeptides was then performed. The 240 kd fragment isolated from a preparative gel produced 160, 140, 115, and 92.5 kd bands. The 80 kd and 77 kd fragments produced a 70 kd and 67 kd fragment, respectively.

A Factor VIIIC complex is derived containing the 77 kd and/or 80 kd species and the 92.5 kd polypeptide as a calcium-bridged complex in highly purified form. The purity of Factor VIIIC material (the complex and precursor species) is usually greater than 80%, often greater than 90 %, and may be 98 % or higher, based on total protein, and that of the complex is at least 20 %, more usually 30 %, based on total protein, following the anti-Factor VIIIR immunosorbent and aminohexyl Sepharose columns. The use of additional chromatographic steps, e.g., using dextran sulfate, increases the level of purity to at least 90% and usually greater, for the Factor VIIIC material (complex plus precursor). The purity of Factor VIIIC components, 92.5 kd species and the 77/80 kd doublet, isolated by preparative SDS gel electrophoresis is usually at least 98 %. As indicated above, the complex can be obtained using monoclonal antibodies specific for a member of the doublet, or for the 92.5 kd polypeptide. The complex may then be separated from the antibody using a denaturing or chaiotropic solution, e.g., aqueous urea or thiocyanate, respectively.

Sequences of these peptides are described in detail in the Experimental section hereinafter.

In accordance with the subject invention, the purified composition of subunits/fragments of Factor VIIIC may be used to enhance clotting capability for individuals requiring the particular subunits. The composition may also be used in therapy. In addition, the composition prepared according to this invention can be used for the production of monoclonal antibodies to Factor VIIIC, its subunits and fragments. Also, the subunits and fragments may be used as reagents, which may be labeled and, in combination with the antibodies, employed in diagnostic assays for the presence of one or more subunits or degradation fragments thereof in physiological fluids, e.g., blood or serum.

The following examples are offered by way of illustration and not by way of limitation.

Whenever used hereinafter Ab stands for antibody, and Ag for antigen.

EXPERIMENTAL

I. Purification of Factor VIIIC

Human Factor VIIIC was isolated from commercial cryoprecipitate preparations by a) immunosorbent chromatography using a polyclonal anti-VIIIR-Sepharose column by a method first described by Tuddenham et al., loc. cit., and b) a chromatographic separation on aminohexyl-substituted agarose as was originally described by Austen, loc. cit.

Details of the procedures are described below.

Goat anti-human Factor VIII Related Antigen (VIII:R) serum obtained from Atlantic Antibody (cat. no. 040-01), was treated with either a standard 0-50 % ammonium sulfate cut followed by DEAE cellulose column chromatography, or a similar 0-33 % cut without subsequent chromatography. These materials were then conjugated to CNBr-activated Sepharose CL2B or 4B, respectively (Pharmacia, 17-0140-01 or 17-0430-01), and poured to a column (anti VIII: R-Sepharose column).

"HEMOFIL", a stable, dried preparation of antihemophilic factor (Factor VIII, AHF, AHG) in concentrated form prepared from fresh, normal human plasma, representing about a 40-fold enrichment for Factor VIIIC, was dissolved in the following buffer: 0.02 M imidazole, 0.15 M NaCl, 0.1 M lysine-HCl, 0.02 % $NaN_3$, pH 7.4.

After being dissolved, the Hemofil was applied to the above-described anti VIII:R-Sepharose column. Non-specifically bound protein was eluted with the above buffer modified to 0.5 M NaCl. Next, Factor VIIIC was eluted with the above buffer containing 0.35 M $CaCl_2$, with the addition of 10 % glycerol which stabilizes the Factor VIIIC activity. Active fractions from the immunosorbent column were pooled and dialysed against buffer (0.02 M imidazole, 0.15 M NaCl, 0.1 M lysine-HCl, 0.025 M $CaCl_2$, 0.02 % $NaN_3$, 10 % glycerol, pH 7.4). An aliquot of the dialysed fractions, which contained 1,100 units of Factor VIIIC, was applied to an aminohexyl-Sepharose 4B column (1x6cm) equilibrated with the dialysis buffer described above. Factor VIIIC activity was eluted with the same buffer containing either 0.35 M CaCl$_2$ or 2 M NaCl. The activity was found to be in a volume of 2 ml with 500 units of Factor VIIIC per ml. Subsequent experiments carried out in the same manner provided a recovery of 25 % off the anti VIII:R column and a recovery of approximately 90 % off the aminohexyl column. Alternatively, pooled, dialysed material eluted from the immunosorbent column is first applied to a dextran sulfate (Pharmacia) column (1.5 x 6cm) equilibrated with the above dialysis buffer and eluted with the same buffer. Several minor contaminants, e.g., fibrinogen, fibronectin, IgG, are retained on the column while Factor VIIIC emerges in the eluate which is collected and applied to the aminohexyl-Sepharose column as before.

Both biological, i.e., clotting, and antigenic (cAg) activity were shown to be present in the purified Factor VIIIC, as demonstrated by the subsequent assays indicating a 5,000-fold purification over the 40-fotd concentration in Hemofil. Using a standard commercially available three component kit from General Diagnostics, (APTT, Factor VIII deficient plasma, Verity Normal Citrate) a coagulation assay was carried out and indicated high levels of Factor VIIIC biological activity.

The antibodies employed were derived from inhibitor patients, one with a low titer (LZ) as coating Ab and one with a high titer (HZ) as the labeled Ab. The antibodies were used in two different types of assays. In an RIA assay, the HZ Ab is labeled with $^{125}$I, while in an ELISA assay the HZ Ab is coupled to horseradish peroxidase. Labeling with $^{125}$I of antibody HZ for the RIA was performed in accordance with Hunter, W.M., In:Radioimmunoassay, Weir; D. M., ed., Handbook of Experimental Immunology, 3rd ed., vol. 1, Blackwell Scientific Publications, Oxford, 1978. HRP-HZ conjugation was in accordance with Wilson and Nakane, In:Immunofluorescence and Related Staining Techniques, Knapp et al., eds., Elsevier, North-Holland Biomedical Press, Amsterdam, 1978, pp. 215-224. LZ had an activity of 700 Bethesda Units/ml while HZ had an activity of 1,500 Bethesda Units/ml. The coating antibody (LZ Ab) was diluted to 3.5 µg/ml in 0.1 M NaHCO$_3$, pH 9.8 (RIA), or 0.05 M imidazole, 0.1 M NaCl, 0.01 % Thimerosal, 0.05 % Tween 20, 5 % BSA (ELISA) or, for either method, PBS-CMF (for 1 liter: 200 mg of KCl, 200 mg of KH$_2$PO$_4$, 8.0 g of NaCl, 1.15 g of anhydrous Na$_2$HPO$_4$, pH 7.4), and 1ml was added to each tube (polystyrene) and incubated overnight at room temperature. This solution was removed by suction, and the tubes were washed 3x with 3-3.5 ml 0.15 M NaCl or PBS-CMF containing 0.05 % Tween 20. Samples or standards (General Diagnostics, Verifiy Normal Citrate, catalog no. 34112) were diluted and added to the tubes to a total volume of 0.9 ml per tube and incubated overnight at room temperature (dilutions were made in 0.02 M Tris, 0.15 M NaCl, 5 % BSA, 0.05 % Tween 20, 0.01 % Thimerosal, pH 6.5, for RIA, or 0.05 M imidazole, 0.1 M NaCl, 0.01 % Thimerosal, 0.05 % Tween 20, 5 % BSA for ELISA, or PBS-CMF for either method). The solutions were removed by suction, and the tubes were washed as before. For RIA, 5 x 10$^5$ cpm of $^{125}$I-labeled antibody to Factor VIIIC (HZ) in 600 µl of RIA dilution buffer was added to each tube which was then incubated at 37 °C for 16-18 h; the solutions were then removed, the tubes washed as before and counted in a gamma counter. For ELISA, 0.9 ml peroxidase conjugated anti-Factor VIIIC (HZ) was added to each tube which was then incubated overnight at room temperature; solutions were removed and the tubes washed as before; then 0.9 ml OPD solution (for 100 ml: 0.73 g of citric acid, 1.19 g of disodium hydrogen phosphate, 0.15 g of o-phenylensdiamine, pH 5.0, with 250 µl of 10 % H$_2$O$_2$ added immediately before use were added followed by incubating at room temperature for 30 min in the dark. To stop this reaction, 0.5 ml of 6N HCl (or 0.9 ml of 1M H$_2$SO$_4$) was added to each tube and the OD$_{492}$ read.

II. Structure of the Factor VIIIC Complex

A. Immunoprecipitation Experiments

Gel filtration experiments were carried out with an AcA 44 column on the Factor VIIIC purified material under the following conditions: 0.1 % insulin (as carrier protein for stabilization), 0.25 M CaCl$_2$, 0.01 % Thiomerosal, 0.05 M imidazole, pH 7.2. The Factor VIIIC coagulation and antigenic activities of the eluate were monitored. Two antigenic peaks were observed. One with Factor VIIIC coagulation activity behaved as a complex with an apparent molecular weight of about 460,000 under these conditions (native). The other peak (devoid of coagulation activity) eluted at an observed molecular weight slightly below 67 kd.

When analyzed by standard analytical Lasmmli SDS-gel electrophoresis (Laemmli, Nature (1970) 227, 680-685), various protein species of 240, 160, 140, 115, 92.5, 80 and 77 kd were obtained. The relationship of these proteins to Factor VIIIC was determined by standard immunoprecipitation procedures. In the immunoprecipitation procedure, S. aureus protein A-Sepharose CL4B or polystyrene beads (1/8in, Precision Plastic Ball Co.) coated with affinity purified second antibody (goat anti-mouse IgG or anti-human IgG) were employed to separate antigen-Ab complexes from free $^{125}$I-labeled Factor VIIIC.

The proteins eluted from the affinity column were iodinated and then reacted with antibodies specific for Factor VIIIC. The antibodies were human inhibitor antibodies isolated from hemophilic patients and referred to as anti-Factor VIIIC (Z) and (E) or inhibitor antibody (Z) and (E).

The results indicated that both antibodies reacted with the 77/80 kd doublet. The "E" antibody also reacted strongly with the 240 kd band and gave weak precipitation of several bands (160, 140, 115, 92.5 kd) between the doublet and 240 kd species. The "Z" antibody also precipitated the 92.5 kd and 240 kd proteins. The strong reaction of the "E" antibody with the 240 kd species suggests that this species is a precursor of Factor VIIIC.

The antibody-column purified Factor VIIIC fraction was iodinated and reacted with the human inhibitor antibody both in the presence and in the absence of EGTA (ethyleneglycol-bis(β-aminoethyl ether)-N,N,N',N'-tetaacetic acid). This allows for an investigation of the role of divalent cations, particularly Ca$^{++}$, in the association of the Factor VIIIC polypeptides. It was observed that the inhibitor antibody (E) precipitates the 77/80 kd doublet, as well as higher molecular weight species of 160, 140, 115 and 92.5 kd. The doublet is always among the stronger bands. This immunoprecipitation experiment was done with the polystyrene beads. This procedure results in lower backgrounds, and the labeled IgG in the Factor VIIIC preparation is not precipitated. Inclusion of EGTA results in the loss of the higher molecular weight bands (92.5-160 kd) but has no effect on the amount of doublet precipitated.

A similar experiment utilized Z antibody coupled to Sepharose as an immunosorbent: Purified Factor VIIIC was applied to the column, and after binding <u>via</u> 77/80 kd, the 92.5 kd polypeptide was selectively eluted with EDTA (ethylene diamine tetraacetic acid). The method is used preparatively to fractionate the 92.5 kd species. This immunosorbent column or a similar one are prepared with polyclonal antibodies to Factor VIIIC. When eluted with chaiotropic or denaturing solvents, e.g., thiocyanate or aqueous urea solutions, respectively, rather than EGTA, Factor VIIIC is further purified. These results suggest that the 92.5 kd peptide may be associated noncovalently to the 77/80 kd doublet via a Ca$^{++}$ bridge. The inhibitor antibody appears to interact directly only with the doublet. The higher molecular weight bands (of 115 kd, 140 kd, 160 kd) are probably precursors of 92.5 kd, as indicated by the ability of the monoclonal antibody directed against the 92.5 kd polypeptide to cross-react with the 115 kd, 140 kd and 160 kd polypeptides.

The relationship of various protein species from the affinity column was demonstrated by immunoprecipitation of iodinated, purified Factor VIIIC with monoclonal antibodies prepared according to the method of G. Kohler and C. Milstein (<u>Eur. J. of Immunol.</u>, 6(1975) 511). Balb/c mice were immunized with liquid phase immunoadsorbed Factor VIIIC. Spleen cells ($10^8$) were fused with $10^7$ NSO or NSI mouse myeloma cells. The fusion products were plated into two 96-well microtiter trays. A spleen cell feeder layer was used at $10^4$ cells/well. Colonies were microscopically visible from the fifth day, and the supernatants were assayed every few days using an ELISA assay. The following layers were employed: 1st: Factor VIIIC eluted from the amino hexyl-Sepharose 4B column, as described in Section I above; 2nd: hybridoma cell supernatant; 3rd: horseradish peroxidase (HRP)-labeled goat anti-mouse IgG; 4th: HRP-substrate.

Several classes of monoclonal antibodies were identified, two of which inhibited Factor VIIIC coagulation activity: Class I antibodies reacted with the 80/77 kd doublet and 240 kd polypeptides; and Class II antibodies reacted with proteins of 240, 160, 140, 115, 92.5 kd. Immunoprecipitation of thrombin-digested Factor VIII with Class I monoclonal antibodies indicates that the 70/67 kd doublet produced is derived from 77/80 kd doublet (see below). Immunoprecipitation with Class III monoclonal antibodies indicates that the 160, 140 and 115 kd peptides are precursors of the 92.5 kd peptide. The monoclonal antibodies of Class III further reacted with a 40 kd peptide produced by thrombin digestion of the purified Factor VIIIC material.

An experiment similar to that described above, using EGTA to investigate the role of Ca** ion in the Factor VIIIC complex, was also performed utilizing a monoclonal antibody-based ELISA assay with the following layers: 1st: monospecific anti(mouse IgG); 2nd: Class III monoclonal antibody (anti-92.5 kd); 3rd: purified Factor VIIIC material; 4th: HRP-human inhibitor antibody to 77/80 kd. Addition of EGTA removed bound HRP activity present in the control without chelator. The fact that the Class I and III monoclonal antibodies directed to the 77/80 kd doublet and 92.5 kd proteins, respectively, are each inhibitory to Factor VIIIC coagulation activity indicates that both are essential components of the Factor VIIIC complex.

B. <u>Thrombin Activation of Factor VIIIC</u>

Aminohexyl-concentrated, affinity-purified Factor VIIIC has been activated by thrombin (Boehringer, lot no. 1072302) using two different sets of pH conditions (6.8 and 7.4).

Aliquots were assayed for coagulation activity and, in addition, samples (about 2.5 units each) were TCA precipitated for gel analysis. In the first experiments, the VIIIC activity was initially 46 units/ml. This was diluted to a final concentration of 11.5 units/ml in Factor VIIIC buffer (20 mM imidazole, pH 6.8, 150 mM NaCl, 100 mM lysine, 25 mM CaCl$_2$ and 10 % glycerol). The final concentration of thrombin was 0.12 unit/ml (about 1 unit of thrombin per 100 coagulation units of VIIIC). The results showed that the coagulation activity increases to about 180 units/ml, and then decreases to about 40 units/ml (essentially the starting value) coincidentally with a similar increase and decrease in the amount of the 92.5 kd species. Thus the 92.5kd species is implicated as part of the active Factor VIIIC complex.

Additional experiments with more concentrated Factor VIIIC preparations were carried out for the purpose of using thrombin activation in a preparative manner. To generate 92.5 kd polypeptide, thrombin was added to the purified Factor VIIIC material (pH 7.4) at a ratio of about 1000-2000 coagulation units of Factor VIIIC to 1 unit of thrombin activity and allowed to react for only a short period of time (5-15 min, depending on the Hemofil sample). The resulting product was then applied to a 7.5 % preparative gel, and the peptides were separated by electrophoresis, the gel bands cut out and electroeluted.

When thrombin digestion is carried out for a short time, the amount of 92.5 kd species can be doubled or tripled; at the same time, the 77/80 kd doublet is only partially converted into 67/70 kd species. To optimize the conditions for the isolation of the 67/70 kd doublet, a longer time course (more than 1 h) thrombin digestion is carried out. In this case, the 92.5 kd species is further cleaved to produce smaller fragments. Two new peptides, 52.5 kd and 40 kd, appear after thrombin treatment. The 40 kd peptide reacts with the monoclonal antibody directed against the 92.5 kd species which must therefore be a cleavage product. The 52.5 kd peptide is also derived from the 92.5 kd protein as demonstrated by a comparison of chemical and enzymatic cleavage patterns, i.e., both the 92.5 kd and 52.5 kd species when subjected to CNBr or endoproteinase lys C cleavage show a number of common fragments (by SDS-PAGE).

For endoproteinase lys C digestion, a weight ratio of lys C to protein of from about 1:1-100, usually 1:10, is used. In the subject digestion, 20 pmol (4.8 μg) of lys C were combined with 200 pmol (14 μg) of 70 kd polypeptide in about 100 μl 0.025 M Tris-HCl, pH 7.7, 0.001 M EDTA, 0.08 % SDS, and the mixture was incubated at 37 °C for 6 h to overnight for complete digestion. Native polyacrylamide gels according to Orstein, Ann. N.Y. Acad. Sci. 121 (1964) 321-349, were used for isolation of lys C digestion products.

C. Thrombin Digestion of Gel Isolated VIIIC-related Proteins

In order to confirm the precursor-product relationship of these peptides, a number of the bands were isolated by preparative SDS gel electrophoresis, etectroeluted and subjected to thrombin digestion. The results were as follows:

1. The 240 kd protein produced muldple bands including 160, 140, 115, 92.5 kd but nothing smaller than 92.5 kd, i.e., no 77/80 kd or 67/70 kd doublet. In addition, a time course for digestion was carried out with the 240 kd fragment and analyzed for gel electrophoresis pattern, coagulation activity, and Factor VIIIC antigenic (cAg) activity. The gel chromatography results were the same as above, and essentially no cAg or coagulation activity was recovered.

2. The 160 kd and 92.5 kd gel-isolated polypeptides do not appear to be substrates for thrombin after isolation from the gel.

3. Thrombin specifically cleaves the gel-isolated 77 kd and 80 kd species to produce new polypeptides of 67 kd and 70 kd, respectively. After thrombin treatment, monoclonal antibodies of Class I precipitate not only the 77/80 kd doublet, but also these 67 and 70 kd species.

D. Amino Acid Sequence Analysis

Partial amino acid sequence information was obtained by standard procedures for the 67/70 kd peptides, the 77/80 kd peptides and the 52.5 kd peptide isolated by preparative SDS electrophoresis. The electrophoretic analysis, together with the amino acid sequence results, indicated that the gel-isolated 77/80 kd, 67/70 kd, 92.5 kd and 52.5 kd polypeptides were obtained at >95 %, usually 98 %, purity. The gel isolated peptides were applied to a gas phase protein sequencer (Applied Biosystems). The PTH-amino acids were applied to an HPLC column (IBM cyano, 25 cm), and the amino sequence was determined from the resulting chromatograms.

The following sequence was determined for the 67/70 kd doublet at its amino terminus:

| 1 | 2 | 3 | 4 | 5 | 6 | 7 | 8 | 9 | 10 | 11 |
|---|---|---|---|---|---|---|---|---|----|----|
| ? | Phe | Gln | Lys | Lys | Thr | Arg | His | Tyr | Phe | Ile |

| 12 | 13 | 14 | 15 | 16 | 17 | 18 | 19 | 20 | 21 | 22 |
|----|----|----|----|----|----|----|----|----|----|----|
| Ala | Ala | Val | Glu | Arg | Leu | Trp | Asp | Tyr | Gly | Met |

For the 52.5 kd protein, which, as shown below, is derived from the 92.5 kd protein, the following amino acid sequence at the N-terminus was determined:

```
1     2     3     4     5     6     7     8     9     10    11    12    13
Ala   Thr   Arg   Arg   Tyr   Tyr   Leu   Gly   Ala   Val   Glu   Leu   Ser

14    15    16    17    18    19    20    21    22    23    24    25    26
Trp   Asp   Tyr   Met   Gln   Ser   Asp   ?     Gly   Glu   Leu   Pro   Val
```

The amino acid sequences of two peptides obtained by digestion of the 77/80 kd doublet with endoproteinase LysC (Boehringer-Mannheim) were determined. The digestion was performed as follows. The 77/80 kd doublet was electrophoresed on an acrylamide protein gel, and bands corresponding to the doublet were electroeluted. The separated material was purified and digested with endoproteinase LysC, and the resulting peptides were separated by reverse phase HPLC. The fractions corresponding to peaks of absorbance at 280 nm were sequenced using an automated sequencer (Applied Biosystems, Foster City, CA, Model A70A). The first sequence was as follows:

```
1     2     3     4     5     6     7     8     9     10    11    12
Tyr   Ala   Ala   Thr   Ser   Gln   Val   Leu   Leu   Pro   Ser   Lys
```

The second peptide had the following sequence:

```
1     2     3     4     5     6     7     8     9     10
Val   Thr   Gly   Val   Thr   Thr   Gln   Gly   Val   Lys
```

The 77/80 kd doublet was also digested with trypsin. The doublet material was purified as described for digestion with endoproteinase LysC, above. Lysines were blocked by citraconylation to allow digestion only at arginines. Citraconylation was performed by suspending the proteins in a denaturing buffer, reducing and carboxymethylating the suspended proteins, and treating with citraconic anhydride while maintaining a pH between 8.5 and 9.0. After citraconylation, the proteins were digested with trypsin, and the resulting peptides separated by reverse phase HPLC. The fractions corresponding to peaks of absorbance at 280 nm were sequenced, as above. The sequence was as follows:

```
1     2     3     4     5     6     7     8     9     10    11    12
Met   Gly   Val   Leu   Gly   Cys   Glu   Ala   Gln   Asp   Leu   Tyr
```

When procedures to determine the N-terminal sequences of the 80 and 77 kd species were carried out, it was found that their amino termini were blocked. Therefore, the N-termini sequence was determined from material obtained by an alternative purification method which included immunoaffinity chromatography and ion exchange chromatography and precluded the use of preparative SDS-polyacrylamide gel electrophoresis. Purification of the 80/77 kd doublet involved application of Factor VIIIC concentrate to a monoclonal antibody column followed by chromatography on a mono S cation exchanger. This material had unblocked N-termini, indicating that the blockage detected in gel purified 80/77 kd was an artifact resulting from the gel electrophoresis. The amino terminal sequence determined for both the 80 and 77 kd species is the following

```
1     2     3     4     5     6     7     8     9     10
Glu   Ile   Thr   ?     ?     ?     Leu   Gln   ?     Asp
11    12    13    14    15    16    17    18    19    20
Gln   Glu   Glu   Ile   Asp   Tyr   ?     Asp   Asp   Ile
```

E. <u>Amino Acid Composition</u>

The amino acid compositions for the 77/80 kd doublet peptides were determined by standard methods to be as follows:

| Amino Acid | 80 kd | 77 kd |
|---|---|---|
| Asp | 58 | 54 |
| Glu | 74 | 76 |
| Cys | 12 | 14 |
| Ser | 47 | 44 |
| Gly | 51 | 46 |
| His | 8 | 12 |
| Arg | 32 | 29 |
| Thr | 35 | 29 |
| Ala | 35 | 33 |
| Pro | 33 | 30 |
| Tyr | 25 | 25 |
| Val | 46 | 44 |
| Met | 17 | 17 |
| Ile | 33 | 35 |
| Leu | 49 | 48 |
| Phe | 32 | 31 |
| Lys | 47 | 41 |
| Total No. Amino Acids | 634 | 608 |
| Calculated Molecular Weight: | 82 kd | 79 kd |

**Claims**

**Claim for the following Contracting States : BE, CH, DE, FR, GB, IT, LI, LU, NL, SE**

1. An isolated protein composition comprising:

    a complex of a 77/80 kD doublet polypeptide fragment calcium bridged with a 92.5 kD polypeptide fragment; and precursor species;
    said complex exhibiting a coagulation activity similar to that of human Factor VIIIC;

    wherein said complex and precursor species have a purity of at least 90% based on total protein, and wherein the complex has a purity of at least 30% based on total protein.

**Claim for the following Contracting State : AT**

1. A method for the preparation of an isolated protein composition, said composition comprising a complex of a 77/80 kD doublet polypeptide fragment calcium bridged with a 92.5 kD polypeptide fragment; and precursor species;

    said complex exhibiting a coagulation activity similar to that of human Factor VIIIC;

    wherein said complex and precursor species have a purity of at least 90% based on total protein, and wherein the complex has a purity of at least 30% based on total protein, said method being **characterised by**

    (A) subjecting human Factor VIIIC to immunosorbent chromatography using a polyclonal anti-VIIIR-Sepharose column,

(B) separating the resulting material on an amino-hexyl-substituted agarose column, and
(C) further chromatographic purification.

## Patentansprüche

### Patentanspruch für folgende Vertragsstaaten : BE, CH, DE, FR, GB, IT, LI, LU, NL, SE

1. Isolierte Proteinzusammensetzung, umfassend einen Komplex aus einem 77/80 kD-Duplett-Polypeptidfragment, das mit einem 92,5 kD-Polypeptidfragment calciumverbrückt ist, und Vorläuferspecies,
wobei der Komplex eine dem Human-Faktor VIIIC ähnliche Koagulationswirkung aufweist,
wobei der Komplex und Precursorspecies eine Reinheit von mindestens 90%, bezogen auf Gesamtprotein, aufweisen und
wobei der Komplex eine Reinheit von mindestens 30%, bezogen auf Gesamtprotein, aufweist.

### Patentanspruch für folgenden Vertragsstaat : AT

1. Verfahren zur Herstellung einer isolierten Proteinzusammensetzung, wobei die Zusammensetzung einen Komplex aus einem 77/80 kD-Duplett-Polypeptidfragment, das mit einem 92,5 kD-Polypeptidfragment calciumverbrückt ist, und Vorläuferspecies umfasst,
wobei der Komplex eine dem Human-Faktor VIIIC ähnliche Koagulationswirkung aufweist,
wobei der Komplex und Precursorspecies eine Reinheit von mindestens 90%, bezogen auf Gesamtprotein, aufweisen und
wobei der Komplex eine Reinheit von mindestens 30%, bezogen auf Gesamtprotein, aufweist, wobei das Verfahren durch

(A) Unterwerfen von Human-Faktor VIIIC einer Immunsorptionschromatographie unter Verwendung einer Sepharosesäule mit polyklonalen anti-VIIIR-Antikörpern,
(B) Abtrennen des resultierenden Materials an einer Säule mit Aminohexylsubstituierter Agarose und
(C) weitere chromatographische Reinigung gekennzeichnet ist.

## Revendications

### Revendication pour les Etats contractants suivants : BE, CH, DE, FR, GB, IT, LI, LU, NL, SE

1. Composition protéique isolée comprenant :

un complexe d'un doublet de fragments de polypeptides 77/80 kD lié par un pont calcium à un fragment polypeptidique de 92,5 kD ; et une espèce précurseur ;
ledit complexe présentant une activité coagulante similaire à celle du facteur VIIIC humain ;

dans laquelle ledit complexe et ladite espèce précurseur présentent une pureté d'au moins 90 % sur la base de la teneur totale en protéines, et
dans laquelle le complexe présente une pureté d'au moins 30 % sur la base de la teneur totale en protéines.

### Revendication pour l'Etat contractant suivant : AT

1. Procédé de préparation d'une composition protéique isolée, ladite composition comprenant un complexe d'un doublet de fragments de polypeptides 77/80 kD lié par un pont calcium à un fragment polypeptidique de 92,5 kD ; et une espèce précurseur ;
ledit complexe présentant une activité coagulante similaire à celle du facteur VIIIC humain ;
dans laquelle ledit complexe et ladite espèce précurseur présentent une pureté d'au moins 90 % sur la base de la teneur totale en protéines, et

dans laquelle le complexe présente une pureté d'au moins 30 % sur la base de la teneur totale en protéines, ledit procédé étant **caractérisé par** les étapes consistant à :

(A) soumettre un échantillon de facteur VIIIC humain à une chromatographie par immunoadsorption en utilisant une colonne anti-VIIIR polyclonal sur gel sépharose,
(B) séparer le matériau résultant sur une colonne sur gel d'agarose substituée par amino-hexyle, et
(C) effectuer une purification chromatographique supplémentaire.